# FASCICULE DE BREVET EUROPEEN

(11) **EP 1 112 045 B1**
(45) Date de publication et mention de la délivrance du brevet: **23.06.2004**
(21) Numéro de dépôt: 99941709.0
(22) Date de dépôt: 07.09.1999
(51) Int. Cl.: A61F 2/16

(54) **IMPLANT INTRA-OCULAIRE DE CHAMBRE ANTERIEURE**
VORDERKAMMERIMPLANTAT
INTRAOCULAR IMPLANT FOR ANTERIOR CHAMBER

(30) Priorité: 07.09.1998 FR 9811159
(43) Date de publication de la demande: 04.07.2001
(73) Titulaire: IOLTECH SA, 17000 La Rochelle (FR); Novartis Pharma GmbH, 1230 Wien (AT)
(72) Inventeur: BAIKOFF, Georges, 13007 Marseille (FR)
(74) Mandataire: Lewitter, Herbert
(86) Numéro de dépôt international: PCT/FR1999/002126
(87) Numéro de publication internationale: WO 2000/013614

(56) Documents cités:
- WO-A-95/28897
- WO-A-98/09585
- GB-A- 2 153 688
- GB-A- 2 171 912
- US-A- 4 404 694
- US-A- 5 207 708
- US-A- 5 769 890

## Description

L'invention concerne un implant intra-oculaire de chambre antérieure, pour le traitement des amétropies de l'oeil phaque. Un implant de ce type comprend une optique, constituée par une lentille circulaire dont le plan moyen est perpendiculaire à l'axe optique de l'oeil, et une haptique constituée par une armature, à anses flexibles, logées dans l'angle irido-cornéen, destinée à fixer et à maintenir, la position de l'optique dans la chambre antérieure. Par «flexible » il faut entendre une structure semi-rigide capable d'assurer à l'ensemble optique + haptique une stabilité permanente dans la chambre antérieure. Cependant cette flexibilité n'est pas telle qu'elle autorise le pliage de l'haptique. De tels implants sont décrits dans les documents FR-2 631 228 et FR 2 666 220. Du fait de leur structure rigide au niveau de l'optique, ces implants exigent, pour leur introduction dans la chambre antérieure de l'oeil, un orifice d'insertion assez grand qui doit ensuite être suturé.

Implants intra-oculaire constitués en matériau flexible susceptible d'être introduits par un orifice de préférence de petite taille, sont connus comme décrits dans les brevets sous les nos. WO 98/09585 et US 5,207,708.

Un but de l'invention est de proposer un implant intra-oculaire susceptible d'être introduit dans la chambre antérieure par un orifice assez petit pour ne pas exiger de suture.

Par ailleurs, les implants des documents FR 2 631 228 et FR 2666 220 ont une haptique qui présente une certaine symétrie.

Cette haptique comporte en général quatre points d'appui dans l'angle irido-coméen, selon une disposition qui doit être essentiellement symétrique. A la mise en place de l'implant, cette symétrie doit être respectée, ce qui allonge parfois la durée de l'intervention chirurgicale.

Un autre but de l'invention est de proposer un implant intra-oculaire qui ne soit pas soumis à cette contrainte de symétrie.

L'invention a pour objet un implant intra-oculaire de chambre antérieure, composé d'une optique circulaire et d'une haptique flexible pour le maintien de l'optique dans la chambre antérieure, les points d'appui de l'haptique étant logés dans l'angle irido-cornéen; l'optique est constituée par une lentille souple susceptible d'être roulée ou pliée lors de l'insertion de l'implant dans la chambre antérieure ; l'haptique présente une zone de liaison avec l'optique, et dans cette zone de liaison, la partie de l'haptique liée à l'optique est située sur le bord de l'optique ; et, dans la zone de liaison. la liaison entre l'optique et l'haptique est rigide de sorte que, lorsque l'implant est en place. l'optique soit maintenue de façon stable dans un plan perpendiculaire à l'axe optique de l'oeil.

Selon d'autres caractéristiques :
- dans la zone de liaison, l'haptique comporte une partie rigide pour assurer la liaison rigide avec l'optique ;
- l'haptique a la forme générale du chiffre 2 et présente trois points d'appui, un au coude et un à chacune des extrémités libres ;
- la zone de liaison est située sur l'anse courbe reliant le coude de l'haptique à l'extrémité libre supérieure ;
- la zone de liaison est située du côté concave de l'anse de l'haptique ;
- la zone de liaison est une zone de tangence entre l'optique et l'haptique ;
- l'haptique présente une sorte de berceau dont le bord est sensiblement circulaire pour être en contact avec la lentille sur une zone de liaison étendue ;
- l'optique est constituée par une lentille en matériau acrylique, en silicone ou en HEMA;
- l'haptique est constituée en PMMA, ou tout autre matériau compatible avec l'oeil.

D'autres caractéristiques ressortent de la description qui suit faite avec référence aux dessins annexés dans lesquels :
- la figure 1 est une vue de face d'un implant selon un mode de réalisation de l'invention ;
- la figure 2 est une vue en coupe axiale simplifiée de la chambre antérieure d'un oeil équipé de l'implant de la figure 1 ;
- la figure 3 est une vue partielle de face de l'implant de la figure 1 ;
- la figure 4 est une vue analogue à celle de la figure 3, d'un autre mode de réalisation de l'implant selon l'invention.

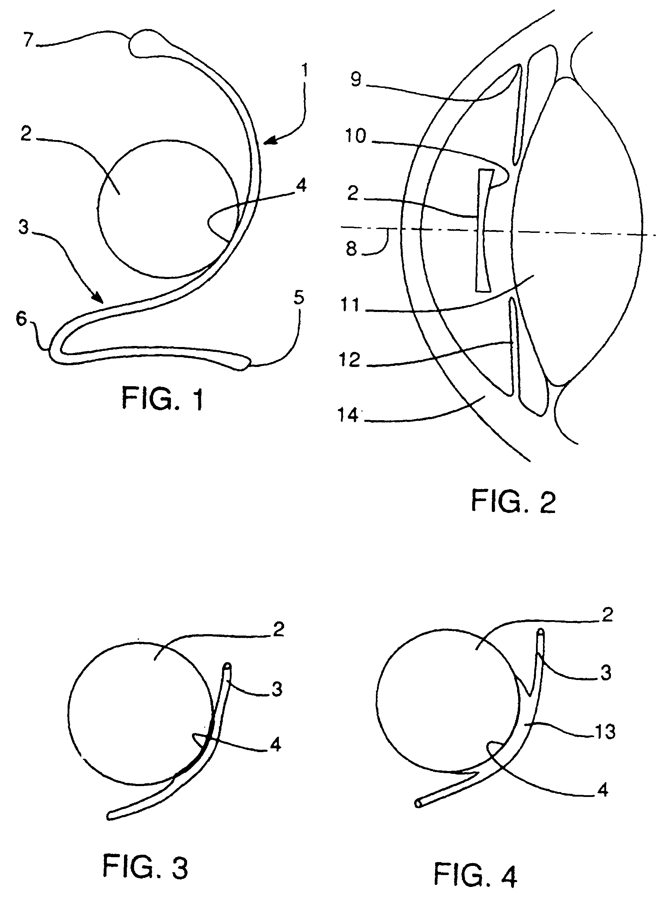

Sur la figure 1, l'implant 1 selon l'invention est constitué d'une optique 2 en forme de lentille circulaire. et d'une haptique 3 ayant la forme générale du chiffre 2.

L'haptique présente trois points de contact avec l'angle irido-cornéen : le premier à son extrémité libre inférieure 5, le deuxième à son coude 6, et le troisième à son extrémité libre supérieure 7. Dans la partie centrale de l'anse courbe reliant le coude 6 à l'extrémité libre supérieure 7 de l'haptique, est prévue une zone de liaison 4 entre l'haptique 3 et l'optique 2.

Dans la zone de liaison 4, la liaison entre l'optique 2 et l'haptique 3 est rigide de sorte que, lorsque l'implant 1 est en place, l'optique 2 soit maintenue de façon stable dans un plan perpendiculaire à l'axe optique de l'oeil. Pour assurer cette rigidité de la liaison, l'haptique 3 comporte avantageusement une partie rigide dans la zone de liaison 4.

Cette stabilité d'orientation de la partie optique dans le plan perpendiculaire à l'axe optique est indispensable non seulement à la qualité optique mais également au respect des structures oculaires voisines (iris, cristallin, cornée) qui pourraient être endommagées si la partie optique pouvait débattre librement d'avant en arrière par rapport à la partie haptique. Ce risque serait très élevé en raison des changements permanents de la position du globe oculaire.

Lorsque l'implant 1 est en place, dans la chambre antérieure de l'oeil (figure 2), l'optique 2 se trouve dans un plan perpendiculaire à l'axe optique 8 de l'oeil, et les trois points de contact 5, 6, 7 de l'haptique se trouvent répartis sur la circonférence de l'angle irido-coméen 9. De préférence, l'optique 2 comporte une lentille dont la face arrière 10 est concave. Cette face concave 10 est tournée vers le cristallin 11 et l'iris 12 de l'oeil. La liaison entre l'optique 2 et l'haptique 3 est réalisée dans la zone de liaison 4, par tout moyen approprié tel que collage, soudage, liaison physico-chimique, ou jonction mécanique.

La lentille de l'optique 2 est disposée du côté concave de l'anse de l'haptique, de façon que la zone de liaison 4 (figure 3) soit une zone de tangence entre l'optique 2 et l'haptique 3.

Selon l'exemple de réalisation de la figure 4, l'haptique présente une sorte de berceau 13 dont le bord est sensiblement circulaire pour être en contact avec la lentille sur une zone de liaison 4 étendue.

Dans la zone de liaison 4, la portion de l'haptique 3 liée à l'optique 2 est située sur le bord de l'optique 2 par exemple dans le plan moyen du bord de la lentille. En dehors de la zone de liaison 4, l'haptique 3 peut présenter des inclinaisons par rapport au plan moyen de la lentille de façon à assurer la position décalée de l'optique 2 par rapport au plan moyen de l'angle irido-cornéen 9 (figure 2). Cette disposition permet d'assurer que la zone de liaison 4, lorsque l'implant 1 est en place, se trouve dans un plan perpendiculaire à l'axe optique 8 de l'oeil, et maintienne l'optique 2 perpendiculairement à cet axe optique 8, en avant du plan irien.

Selon une caractéristique essentielle de l'invention, l'optique 2 est constituée par une lentille souple, susceptible, pour l'insertion de l'implant 1 dans la chambre antérieure, d'être roulée sur elle-même ou autour de l'haptique dans la zone de liaison 4, l'axe d'enroulement étant parallèle à la direction moyenne de l'haptique dans la zone de liaison 4.

La lentille peut également être repliée autour d'un axe parallèle à la direction moyenne de l'haptique dans la zone de liaison 4. Après insertion de l'implant, la lentille souple est libérée et elle reprend sa forme initiale.

Pour assurer cette souplesse et cette élasticité à la lentille, le matériau constitutif de la lentille est par exemple un matériau acrylique, de la silicone, ou de l'HEMA.

Le matériau constitutif de l'haptique 3 est flexible mais suffisamment rigide pour maintenir l'optique 2 en position stable : le PMMA est utilisé de préférence.

L'insertion de l'implant 1 dans la chambre antérieure s'effectue par un orifice d'insertion pratiqué dans la cornée 14. Comme la lentille souple est roulable contre, ou sur, l'anse de l'haptique 3, ou pliable sur l'anse de l'haptique 3, la plus grande dimension de l'orifice d'insertion est inférieure ou égale à 4 mm, de sorte que cet orifice d'insertion est auto-étanche et n'a pas besoin d'être suturé, ce qui simplifie l'intervention chirurgicale.

L'introduction de l'implant par l'orifice d'insertion s'effectue par l'extrémité inférieure 5 de l'haptique, puis par le coude 6, puis par l'anse de l'haptique sur laquelle, ou contre laquelle, est roulée ou pliée la lentille de l'optique 2, et enfin par l'extrémité libre supérieure 7.

L'implant selon l'invention peut être utilisable pour le traitement d'une amétropie, par exemple la myopie, l'hypermétropie ou l'astigmatisme. Il est seulement nécessaire que le bord de l'optique soit assez épais pour assurer la liaison avec l'haptique dans la zone de liaison 4. Cette condition est plus particulièrement facile à réaliser dans le cas de la myopie car la lentille est alors à bord épais.

A titre d'exemple, un implant tripode intra-oculaire de chambre antérieure selon l'invention est destiné à être logé dans le cercle correspondant à l'angle irido-cornéen qui a un diamètre inférieur ou égal à 13,5 mm. Le diamètre de l'optique 2 et inférieur ou égal à 6mm. L'anse de l'haptique 3 a une épaisseur d'environ 0,1mm. La plus grande dimension de l'orifice d'insertion de l'implant dans la chambre antérieure est inférieure ou égale à 4 mm et cet orifice est non suturable.

## Revendications

1. Implant intra-oculaire de chambre antérieure pour le traitement des amétropies de l'oeil phaque, composé d'une optique circulaire constituée par une lentille souple susceptible d'être roulée ou pliée lors de l'insertion de l'implant (1) dans la chambre antérieure et d'une haptique flexible pour le maintien de l'optique dans la chambre antérieure, les points d'appui de l'haptique étant logés dans l'angle irido-cornéen,
- l'haptique (3) ayant la forme générale du chiffre 2 et présentant trois points d'appui, un au coude (6) et un à chacune des extrémités libres (5, 7);
- l'haptique (3) présentant une zone de liaison (4) avec l'optique (2), la liaison étant réalisée par collage, soudage, liaison physico-chimique ou jonction mécanique, et dans cette zone de liaison (4), la partie de l'haptique (3) liée à l'optique (2) étant située sur le bord de l'optique (2);
- dans la zone de liaison (4), la liaison entre l'optique (2) et l'haptique (3) étant rigide de sorte que, lorsque l'implant (1) est en place, l'optique (2) soit maintenue de façon stable dans un plan perpendiculaire à l'axe optique de l'oeil;
- la zone de liaison (4) étant située sur une anse courbe reliant le coude (6) de l'haptique (3) à l'extrémité libre supérieure (7);
- la zone de liaison (4) étant située du côté concave de l'anse de l'haptique (3); et
- la zone de liaison (4) étant une zone de tangence entre l'optique (2) et l'haptique (3).

2. Implant selon la revendication 1, **caractérisé en ce que**, dans la zone de liaison (4), l'haptique (3) comporte une partie rigide pour assurer la liaison rigide avec l'optique (2).

3. Implant selon la revendication 1, **caractérisé en ce que** l'haptique (3) présente une sorte de berceau (13) dont le bord est sensiblement circulaire pour être en contact avec la lentille sur une zone de liaison (4) étendue.

4. Implant selon la revendication 1, **caractérisé en ce que** l'optique (2) est constituée par une lentille en matériau acrylique, en silicone ou en HEMA.

5. Implant selon la revendication 1, **caractérisé en ce que** l'haptique (3) est constituée en PMMA, ou tout autre matériau compatible avec l'oeil.

## Patentansprüche

1. Intraokulares Vorderkammerimplantat für die Behandlung der Fehlsichtigkeit des phaken Auges, zusammengesetzt aus einer kreisförmigen Optik, gebildet durch eine weiche Linse, die geeignet ist, beim Einsetzen des Implantates (1) in die Vorderkammer gerollt oder gefaltet zu werden, und ein flexibles Kontaktteil für das Halten der Optik in der Vorderkammer, wobei die Stützpunkte des Kontaktteils in dem Irido-Cornea-Winkel aufgenommen werden,
- das Kontaktteil (3) besitzt die allgemeine Form der Ziffer 2 und weist drei Stützpunkte auf, einen an der Krümmung (6) und einen an jedem der freien Enden (5; 7);
- das Kontaktteil (3) weist eine Zone (4) der Verbindung mit der Optik (2) auf, wobei die Verbindung durch Kleben, Verschweißen, physikalisch-chemische Bindung oder mechanische Verbindung realisiert wird, und in dieser Verbindungszone (4) befindet sich der mit der Optik (2) verbundene Teil des Kontaktteils (3) am Rand der Optik (2);
- in der Verbindungszone (4) ist die Bindung zwischen der Optik (2) und dem Kontaktteil (3) starr, so daß in dem Fall, wo das Implantat (1) eingesetzt wird, die Optik (2) in stabiler Weise in einer Ebene senkrecht zur optischen Achse des Auges gehalten wird;
- die Verbindungszone (4) befindet sich in einem Kurvenbogen, der die Krümmung (6) des Kontaktteils (3) mit dem freien oberen Ende (7) verbindet;
- die Verbindungszone (4) befindet sich an der konkaven Seite des Bogens des Kontaktteils (3); und
- die Verbindungszone (4) ist eine Zone der Berührung zwischen der Optik (2) und dem Kontaktteil (3).

2. Implantat nach Anspruch 1, **dadurch gekennzeichnet, daß** in der Verbindungszone (4) das Kontaktteil (3) einen starren Teil umfaßt, um die starre Verbindung mit der Optik (2) zu gewährleisten.

3. Implantat nach Anspruch 1, **dadurch gekennzeichnet, daß** das Kontaktteil (3) eine Art Wiege (13) aufweist, deren Rand etwa kreisförmig ist, um über eine ausgedehnte Verbindungszone (4) in Kontakt mit der Linse zu treten.

4. Implantat nach Anspruch 1, **dadurch gekennzeichnet, daß** die Optik (2) durch eine Linse aus acrylischem Material, aus Silicon oder aus HEMA gebildet wird.

5. Implantat nach Anspruch 1, **dadurch gekennzeichnet, daß** das Kontaktteil (3) aus PMMA besteht oder aus jedem anderen Material, das mit dem Auge kompatibel ist.

## Claims

1. Anterior chamber intraocular implant for treating ametropias of the phakic eye, composed of a circular optic part, consisting of a flexible lens which can be rolled or folded upon insertion of the implant (1) into the anterior chamber, and of a flexible haptic part for maintaining the optic part in the anterior chamber, the bearing points of the haptic part being lodged in the iridocomeal angle,
- the haptic part (3) having the general shape of the number 2 and having three bearing points, one at the bend (6) and one at each of the free ends (5, 7);
- the haptic part (3) having a zone of connection (4) to the optic part (2), the connection being made by bonding, welding, physicochemical connection or mechanical joining, and in this connection zone (4) the portion of the haptic part (3) connected to the optic part (2) being situated on the edge of the optic part (2);
- in the connection zone (4), the connection between the optic part (2) and the haptic part (3) being rigid, so that when the implant (1) is in place, the optic part (2) is maintained stable in a plane perpendicular to the optic axis of the eye;
- the connection zone (4) being situated on the curved loop connecting the bend (6) of the haptic part (3) to the upper free end (7);
- the connection zone (4) being situated on the concave side of the loop of the haptic part (3); and
- the connection zone (4) being a zone of tangency between the optic part (2) and the haptic part (3).

2. Implant according to Claim 1, **characterized in that**, in the connection zone (4), the haptic part (3) comprises a rigid portion in order to ensure rigid connection to the optic part (2).

3. Implant according to Claim 1, **characterized in that** the haptic part (3) has a sort of cradle (13) whose edge is substantially circular so as to be in contact with the lens over an extended connection zone (4).

4. Implant according to Claim 1, **characterized in that** the optic part (2) consists of a lens made of acrylic material, silicone or HEMA.

5. Implant according to Claim 1, **characterized in that** the haptic part (3) is made of PMMA, or of any other material compatible with the eye.
